# EUROPEAN PATENT APPLICATION

(11) **EP 4 585 149 A1**
(43) Date of publication of application: **16.07.2025**
(21) Application number: 24150933.0
(22) Date of filing: 09.01.2024
(51) Int. Cl.: A61B 5/18, A61B 5/00, A61B 5/0205, B60W 40/08, G16H 10/60, A61B 5/024, A61B 5/08, A61B 5/107

(54) **DRIVER ASSISTANCE SYSTEM**

(71) Applicant: Harman Becker Automotive Systems GmbH, 76307 Karlsbad (DE)
(72) Inventor: AMTHOR, Peter, 76307 Karlsbad (DE)
(74) Representative: Westphal, Mussgnug & Partner, Patentanwälte mbB

(57) **Abstract**

A method comprises, at the beginning of each driving session of a vehicle, collecting one or more physiological parameters and one or more physical parameters for each of one or more passengers of the vehicle, for each passenger of the one or more passengers of the vehicle, storing the one or more physiological parameters and one or more physical parameters, thereby creating a temporary personal profile for each passenger, and, if an increased risk for the occurrence of an accident of the vehicle is detected or if it is detected that an accident has occurred, providing the temporary personal profile of each passenger of the one or more passengers of the vehicle to one or more vehicle applications.

## Description

### TECHNICAL FIELD

The disclosure relates to a driver assistance system and related method.

### BACKGROUND

Driver assistance can include any support that is provided to a driver of a vehicle with the aim of increasing personal safety and enhancing driver experience. Driver assistance systems can be used in order to prevent accidents, to reduce the severity of accidents, and to provide help when an accident did happen.

There is a need for a driver assistance system and a related method that can efficiently reduce the severity of accidents and provide help when an accident did happen.

### SUMMARY

A method includes, at the beginning of each driving session of a vehicle, collecting one or more physiological parameters and one or more physical parameters for each of one or more passengers of the vehicle, for each passenger of the one or more passengers of the vehicle, storing the one or more physiological parameters and one or more physical parameters, thereby creating a temporary personal profile for each passenger, and, if an increased risk for the occurrence of an accident of the vehicle is detected or if it is detected that an accident has occurred, providing the temporary personal profile of each passenger of the one or more passengers of the vehicle to one or more vehicle applications.

A driver assistance system includes a processor, wherein the processor is configured to, at the beginning of each driving session of a vehicle, collect one or more physiological parameters and one or more physical parameters for each of one or more passengers of the vehicle, for each passenger of the one or more passengers of the vehicle, store the one or more physiological parameters and one or more physical parameters, thereby creating a temporary personal profile for each passenger, and, if an increased risk for the occurrence of an accident of the vehicle is detected or if it is detected that an accident has occurred, provide the temporary personal profile of each passenger of the one or more passengers of the vehicle to one or more vehicle applications.

Other systems, features and advantages of the disclosure will be or will become apparent to one with skill in the art upon examination of the following detailed description and figures. It is intended that all such additional systems, methods, features and advantages included within this description be within the scope of the invention and be protected by the following claims.

### BRIEF DESCRIPTION OF THE DRAWINGS

The arrangements and methods may be better understood with reference to the following description and drawings. The components in the figures are not necessarily to scale, emphasis instead being placed upon illustrating the principles of the invention. Moreover, in the figures, the same reference numerals designate the same components throughout the different views.
Figure 1 schematically illustrates a driver assistance system according to embodiments of the disclosure in a vehicle.
Figure 2 schematically illustrates a driver assistance system according to embodiments of the disclosure.
Figure 3 schematically illustrates, in a flow diagram, a method according to embodiments of the disclosure.

### DETAILED DESCRIPTION

The driver assistance system and related method according to the various embodiments described herein can efficiently reduce the severity of accidents and provide help when an accident did happen.

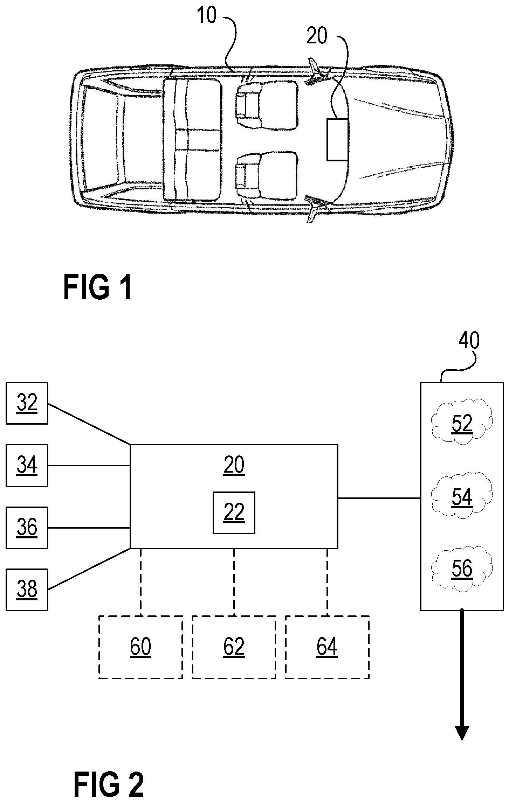

Figure 1 schematically illustrates a vehicle 10 comprising a driver assistance system 20. The driver assistance system 20 is schematically illustrated in further detail in Figure 2. The driver assistance system 20 comprises a processor 22 configured to, at the beginning of each driving session of the vehicle 10, collect one or more physiological parameters and one or more physical parameters for each of one or more passengers of the vehicle 10. For each passenger of the one or more passengers of the vehicle 10, the one or more physiological parameters and the one or more physical parameters are stored, thereby creating a temporary personal profile 52, 54, 56 for each passenger. If an increased risk for the occurrence of an accident of the vehicle 10 is detected, or if it is detected that an accident has occurred, the temporary personal profile 52, 54, 56 of each passenger of the one or more passengers of the vehicle 10 is provided to one or more vehicle applications.

The driver assistance system 20 may comprise or may be coupled to one or more sensors 32, 34, 36, 38 arranged in the vehicle 10. Collecting the one or more physiological parameters and the one or more physical parameters may comprise receiving one or more parameters from the one or more sensors 32, 34, 36, 38. The one or more sensors 32, 34, 36, 38 may comprise at least one of a camera 32, a radar sensor 34, a thermal imaging camera 36, and a weight sensor 38, for example. Any other kind of sensor may be used, with which physiological and physical parameters of a passenger may be determined.

The one or more physiological parameters may include at least one of a heart rate, a respiratory rate, and a body temperature of the respective passenger, for example. The one or more physical parameters may include at least one of a size, a weight, an age, and a gender of the respective passenger, for example. Such physiological and physical parameters related to a specific passenger allow determining a general physique and physical condition of the respective passenger. The physiological and physical parameters can be collected in a very safe and unobtrusive way. The passenger may not even notice that the parameters are determined. For example, a size and weight of a passenger may be determined or at least estimated by means of one or more cameras 32 (e.g., using suitable image processing techniques). One or more cameras are often already present in a vehicle for other applications, e.g., for drowsiness detection applications. The weight of a passenger may alternatively or additionally be determined by means of a weight sensor integrated in the respective vehicle seat, the passenger is seated on. Such sensors are often already present in a vehicle for applications which detect the presence of passengers, in order to output a warning if a passenger does not wear their seatbelt. An age and a gender of a passenger may be determined or at least estimated by means of cameras and suitable image processing techniques, for example.

Physiological parameters such as, e.g., heart rate, respiratory rate, and body temperature can be determined by means of cameras, radar sensors, and thermal imaging cameras, for example, without requiring any specific action of a passenger. A heart rate and respiratory rate may be determined in a very accurate way by means of radar sensors 34, for example, while a thermal imaging camera 36 may be used to determine a body temperature. Any other suitable sensors, however, may alternatively or additionally used to determine physiological parameters.

Many people today have at least one permanent personal profile stored in a cloud or social network 64, for example. Many people today also use wearable devices such as fitness trackers 62 (e.g., smart watches or dedicated fitness tracking wristbands) which require a permanent personal profile to be stored either in the device itself or in a corresponding cloud. Such permanent personal profiles may include physical parameters such as, e.g., age, gender, size, weight, etc. The driver assistance system 20 may be coupled to a fitness tracking device 62 of a passenger, or to a cloud or social network 64 and receive one or more physical parameters from the fitness tracking device 62, cloud or social network 64. Such parameters may be permanently stored in permanent personal profiles, as they most likely do not change significantly over time, or, if parameters such as, e.g., a weight, change, they are usually updated by users accordingly. A fitness tracking device 62 or other wearable sensors 60 such as, e.g., wearable electrocardiogram, ECG, devices, may also provide current physiological parameters such as, e.g., heart rate, respiratory rate, etc., to the driver assistance system 20. The driver assistance system 20 may store parameters received from the one or more sensors 32, 34, 36, 38 arranged in the vehicle, and parameters received from other external devices (e.g., wearable sensor(s) 60, fitness tracking device(s) 62, cloud or social network 64) in the respective personal profile(s) 52, 54, 56. Accessing an external database (e.g., fitness tracking device(s) 62, cloud or social network 64) may require identification of the passenger (e.g., by means of face recognition techniques). Further, a passenger may be required to enter a password of the external database, or a password may already be known to the driver assistance system 20 such that it can access the external database, when necessary.

The one or more physiological parameters and the one or more physical parameters are determined at least once at the beginning of a driving session. For example, the parameters may be determined and a temporary personal profile 52, 54, 56 may be stored when an engine of the vehicle 10 is started. According to another example, the parameters may be determined and a temporary personal profile may be stored when the engine of the vehicle 10 has been started and the vehicle 10 begins to drive. It is, however, generally also possible that the parameters are determined and the temporary personal profile 52, 54, 56 is stored when it is determined that a person is seated on a seat of the vehicle, before the vehicle 10 is even started and/or begins to move.

At least some physiological parameters may change during a driving session. Therefore, it is possible that the temporary personal profile 52, 54, 56 of each passenger of one or more passengers of the vehicle 10 is updated at least once during the driving session. According to one example, the temporary personal profile(s) 52, 54, 56 may be updated in regular intervals, e.g., every 10 minutes, or every 30 minutes. Alternatively or additionally, it is, however, also possible that the temporary personal profile(s) 52, 54, 56 are updated upon occurrence of a triggering event. For example, the temporary personal profile(s) 52, 54, 56 may be updated if an increased risk for the occurrence of an accident of the vehicle 10 is detected. This may be the case, for example, if a distance between the vehicle 10 and a preceding vehicle decreases rapidly or falls below a defined threshold. An increased risk for the occurrence of an accident, however, may be detected in any suitable way.

If an increased risk for the occurrence of an accident of the vehicle 10 is detected or if it is detected that an accident has already occurred, the temporary personal profile 52, 54, 56 of each passenger of the one or more passengers of the vehicle 10 is provided to one or more vehicle applications (indicated by means of an arrow in Figure 2). The one or more vehicle applications may comprise an airbag unit of the vehicle 10, a braking unit of the vehicle 10, a seat belt tightening unit of the vehicle 10, and/or an emergency call unit of the vehicle 10.

If it is detected that an accident is about to happen, for example, an airbag of the vehicle 10 may be adjusted in accordance with the physiological and physical parameters stored in the respective temporary personal profile 52, 54, 56. For example, an airbag may be filled with either more air or less air, depending on a size and weight of a passenger. Additionally, or alternatively, a seat belt of the respective passenger may be preloaded in order to prepare for an accident. The preload of a seat belt may also be determined based on the size and weight of a passenger, for example. The vehicle 10 may also prepare to perform an emergency braking maneuver. Based on a weight of each passenger of the vehicle 10, the overall weight of the vehicle 10 including the passenger(s) may be determined. A braking force may be determined based on the determined overall weight, for example.

If an accident has occurred, some or all of the parameters stored in the temporary personal profile(s) 52, 54, 56 may be provided to an authority, for example. Many vehicles today make an automatic emergency call, when it has been detected that the vehicle 10 was involved in an accident. The physiological and physical parameters may be transmitted as part of such an emergency call. Any medical staff arriving at the scene of the accident would then already be informed about a physical condition of each passengers before the accident. That is, medical staff would have information about passengers that were already in a bad physical condition before the accident and might need treatment more urgently than other passengers.

If the one or more sensors 32, 34, 36, 38 arranged in the vehicle 10 are still functioning after an accident occurred, it is even possible that the temporary personal profile(s) 52, 54, 56 are updated again in order to determine a physical condition of the passenger(s) after the accident occurred. That is, the occurrence of an accident may be a triggering event which causes the temporary personal profile(s) 52, 54, 56 to be updated. It is generally possible that the physiological and physical parameters stored in the temporary personal profile(s) 52, 54, 56 are transmitted to a competent authority as such. The parameter(s) may then be evaluated by the authority, for example. According to one example, however, an emergency report may be created by the driver assistance system 20 by evaluating the one or more physiological parameters and the one or more physical parameters by means of an artificial intelligence, AI. Such an emergency report may then be transmitted to a competent authority.

The driver assistance system may comprise or may be coupled to a storage device 40, wherein the temporary personal profile 52, 54, 56 of each passenger of the one or more passengers of the vehicle 10 is stored in the storage device 40. Any data stored in the storage device 40 may only be accessible by the driver assistance system 20. That is, the physiological and physical parameters of the passenger(s) may not be available to others and may not be used by any other systems. The temporary personal profile(s) 52, 54, 56 may be permanently deleted at the end of each driving session.

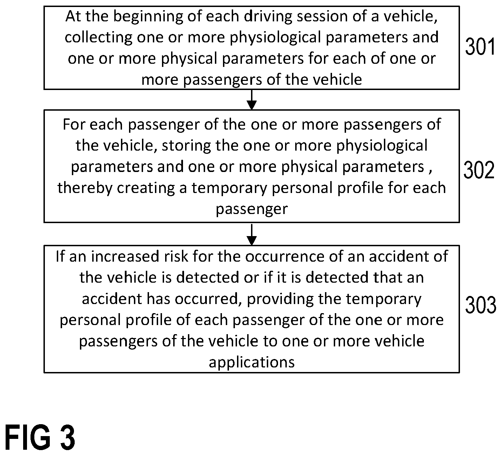

Now referring to Figure 3, a method according to embodiments of the disclosure is schematically illustrated in a flow diagram. The method comprises, at the beginning of each driving session of a vehicle 10, collecting one or more physiological parameters and one or more physical parameters for each of one or more passengers of the vehicle 10 (step 301). For each passenger of the one or more passengers of the vehicle 10, the one or more physiological parameters and one or more physical parameters are stored, thereby creating a temporary personal profile 52, 54, 56 for each passenger (step 302). If an increased risk for the occurrence of an accident of the vehicle 10 is detected or if it is detected that an accident has occurred, the temporary personal profile 52, 54, 56 of each passenger of the one or more passengers of the vehicle 10 is provided to one or more vehicle applications (step 303).

The one or more physiological parameters may include at least one of a heart rate, a respiratory rate, and a body temperature of the respective passenger. The one or more physical parameters may include at least one of a size, a weight, an age, and a gender of the respective passenger. Collecting the one or more physiological parameters and the one or more physical parameters may comprise receiving one or more parameters from one or more sensors 32, 34, 36, 38 arranged in the vehicle 10.

According to one example, collecting the one or more physiological parameters and the one or more physical parameters may further comprise receiving one or more parameters from one or more wearable sensors 60, from one or more fitness tracking devices 62, and/or from one or more permanent personal profiles of the respective passenger in a cloud or social network 64. Providing the temporary personal profile 52, 54, 56 of each passenger of the one or more passengers of the vehicle 10 to one or more vehicle applications may comprise providing the temporary personal profile 52, 54, 56 to an airbag unit of the vehicle 10, to a braking unit of the vehicle 10, to a seat belt tightening unit of the vehicle 10, and/or to an emergency call unit of the vehicle 10.

According to one example, the method may further comprise updating the temporary personal profile 52, 54, 56 of each passenger of the one or more passengers of the vehicle 10 at least once during the driving session. For example, the temporary personal profile 52, 54, 56 of each passenger of the one or more passengers of the vehicle 10 may be updated in regular intervals and/or upon occurrence of a triggering event.

According to one example, an emergency report may be created by evaluating the one or more physiological parameters and the one or more physical parameters by means of an artificial intelligence, AI. An emergency report may be created, for example, when it is detected that an accident has occurred. The emergency report may be transmitted to a competent authority instead of or in addition to the unprocessed physiological and physical parameters.

It may be understood, that the illustrated systems and methods are merely examples. While various embodiments of the invention have been described, it will be apparent to those of ordinary skill in the art that many more embodiments and implementations are possible within the scope of the invention. In particular, the skilled person will recognize the interchangeability of various features from different embodiments. Although these techniques and systems have been disclosed in the context of certain embodiments and examples, it will be understood that these techniques and systems may be extended beyond the specifically disclosed embodiments to other embodiments and/or uses and obvious modifications thereof. Accordingly, the invention is not to be restricted except in light of the attached claims and their equivalents.

The description of embodiments has been presented for purposes of illustration and description. Suitable modifications and variations to the embodiments may be performed in light of the above description or may be acquired from practicing the methods. The described arrangements are exemplary in nature, and may include additional elements and/or omit elements. As used in this application, an element recited in the singular and proceeded with the word "a" or "an" should be understood as not excluding plural of said elements, unless such exclusion is stated. Furthermore, references to "one embodiment" or "one example" of the present disclosure are not intended to be interpreted as excluding the existence of additional embodiments that also incorporate the recited features. The terms "first," "second," and "third," etc. are used merely as labels, and are not intended to impose numerical requirements or a particular positional order on their objects. The subject matter of the present disclosure includes all novel and non-obvious combinations and sub-combinations of the various systems and configurations, and other features, functions, and/or properties disclosed. The following claims particularly point out subject matter from the above disclosure that is regarded as novel and non-obvious.

## Claims

1. A method comprises,
at the beginning of each driving session of a vehicle (10), collecting one or more physiological parameters and one or more physical parameters for each of one or more passengers of the vehicle (10),
for each passenger of the one or more passengers of the vehicle (10), storing the one or more physiological parameters and one or more physical parameters, thereby creating a temporary personal profile (52, 54, 56) for each passenger, and
if an increased risk for the occurrence of an accident of the vehicle (10) is detected or if it is detected that an accident has occurred, providing the temporary personal profile (52, 54, 56) of each passenger of the one or more passengers of the vehicle (10) to one or more vehicle applications.

2. The method of claim 1, wherein the one or more physiological parameters include at least one of a heart rate, a respiratory rate, and a body temperature of the respective passenger.

3. The method of claim 1 or 2, wherein the one or more physical parameters include at least one of a size, a weight, an age, and a gender of the respective passenger.

4. The method of any of claims 1 to 3, wherein collecting the one or more physiological parameters and the one or more physical parameters comprises receiving one or more parameters from one or more sensors (32, 34, 36, 38) arranged in the vehicle (10).

5. The method of claim 4, wherein collecting the one or more physiological parameters and the one or more physical parameters further comprises receiving one or more parameters from one or more wearable sensors (60), from one or more fitness tracking devices (62), and/or from one or more permanent personal profiles of the respective passenger in a cloud or social network (64).

6. The method of any of the preceding claims, wherein providing the temporary personal profile (52, 54, 56) of each passenger of the one or more passengers of the vehicle (10) to one or more vehicle applications comprises providing the temporary personal profile (52, 54, 56) to an airbag unit of the vehicle (10), to a braking unit of the vehicle (10), to a seat belt tightening unit of the vehicle (10), and/or to an emergency call unit of the vehicle (10).

7. The method of any of the preceding claims, further comprising updating the temporary personal profile (52, 54, 56) of each passenger of the one or more passengers of the vehicle (10) at least once during the driving session.

8. The method of claim 7, wherein the temporary personal profile (52, 54, 56) of each passenger of the one or more passengers of the vehicle (10) is updated in regular intervals and/or upon occurrence of a triggering event.

9. The method of any of the preceding claims, further comprising creating an emergency report by evaluating the one or more physiological parameters and the one or more physical parameters by means of an artificial intelligence, AI.

10. A driver assistance system (20) comprises a processor (22) configured to
at the beginning of each driving session of a vehicle (10), collect one or more physiological parameters and one or more physical parameters for each of one or more passengers of the vehicle (10),
for each passenger of the one or more passengers of the vehicle (10), store the one or more physiological parameters and one or more physical parameters, thereby creating a temporary personal profile (52, 54, 56) for each passenger, and
if an increased risk for the occurrence of an accident of the vehicle (10) is detected or if it is detected that an accident has occurred, provide the temporary personal profile (52, 54, 56) of each passenger of the one or more passengers of the vehicle (10) to one or more vehicle applications.

11. The driver assistance system (20) of claim 10, wherein the driver assistance system (20) comprises or is coupled to one or more sensors (32, 34, 36, 38) arranged in the vehicle (10), and wherein collecting the one or more physiological parameters and the one or more physical parameters comprises receiving one or more parameters from the one or more sensors (32, 34, 36, 38).

12. The driver assistance system (20) of claim 11, wherein the one or more sensors (32, 34, 36, 38) comprise at least one of a camera (32), a radar sensor (34), a thermal imaging camera (36), and a weight sensor (38).

13. The driver assistance system of any of claims 10 to 12, further comprising or being coupled to a storage device (40), wherein the temporary personal profile (52, 54, 56) of each passenger of the one or more passengers of the vehicle (10) is stored in the storage device (40).
